# EUROPÄISCHE PATENTSCHRIFT

(11) **EP 1 452 200 B1**
(45) Veröffentlichungstag und Bekanntmachung des Hinweises auf die Patenterteilung: **23.04.2008**
(21) Anmeldenummer: 04012667.4
(22) Anmeldetag: 25.03.2003
(51) Int. Cl.: A61M 25/00, A61M 25/01

(54) **Katheter zur kardiovaskulären Anwendung**
Catheter for cardiovascular use
Cathéter cardio-vasculaire

(30) Priorität: 19.04.2002 DE 10217509
(43) Veröffentlichungstag der Anmeldung: 01.09.2004
(62) Teilanmeldung aus: 03006624.5
(73) Patentinhaber: BIOTRONIK GmbH & Co. KG, 12359 Berlin (DE)
(72) Erfinder: Kolberg, Gernot, 12043 Berlin (DE); Kranz, Curt, Dr., 14163 Berlin (DE); Flach, Erhard, 12305 Berlin (DE)
(74) Vertreter: Lindner-Vogt, Karin L.

(56) Entgegenhaltungen:
- DE-A- 19 930 266
- US-A- 5 167 637
- US-A- 5 228 452
- US-A- 5 356 381
- US-A- 5 376 084
- US-A- 6 024 729

## Beschreibung

Kardiovaskulär anwendbare Katheter umfassen beispielsweise auch Elektroden für Herzschrittmacher oder Defibrillatoren, für die unterschiedlichste Ausführungsformen bekannt sind. Eine besondere Problematik bei solchen Elektroden tritt dann auf, wenn diese nicht nur in den leicht anzusteuernden Atrien oder Ventrikeln des Herzens positioniert, sondern in schwer zugänglichen Herzgefäßen, wie zum Beispiel dem Koronarsinus, eingeführt werden sollen.

Ein Katheter, der zum Einführen in den Koronarsinus oder andere schwer zugängliche Blutgefäße insbesondere bei Einsatz als Herzschrittmacherelektrodensonde geeignet ist, ist aus der DE 199 30 266 A1 bekannt. Dieser Katheter weist einen langgestreckten Katheterschaft mit einem Lumen auf, in dem ein hülsenartiger Mandrin - eine federelastische, relativ formstabile Führungshülse - verläuft. Dieser Mandrin wiederum nimmt einen Führungsdraht auf, der durch eine Schleusenöffnung am distalen Ende des Katheters daraus herausführbar ist.

Dieser Führungsdraht ist vorgebogen und wird als Sonde verwendet, die über die Katheterspitze hinaus vorgeschoben werden kann und aufgrund der Vorbiegung des Drahtes in eine von der Geradeaus-Richtung des Katheters abweisende Richtung weist. Auf diese Weise ist es möglich, mit dem vorgebogenen Draht in eine Abzweigung der Blutgefäße vorzudringen und den dann von dem vorgebogenen Draht geführten Katheter nachzuschieben.

Trotz der erhöhten Flexibilität und damit genaueren Steuerbarkeit dieses Katheters ist er in mehreren Punkten verbesserungsbedürftig. So ist der Katheter lediglich dafür vorgesehen, mit Mandrinhülse und Führungsdraht in das Herz eingeführt zu werden, wobei lediglich an kritischen Punkten der Führungsdraht durch Ausschieben und Lenken in beispielsweise ein Herzgefäß zum Einsatz kommt. Bei einem getrennten Einführen des Führungsdrahtes von außen und einem Nachschieben des Katheters über die gesamte Länge des Führungsdrahtes nach der sogenannten "Over-the-Wire-Technik" besteht das Problem, dass durch die Schleusendichtung an der distalen Durchtrittsöffnung für den Führungsdraht dieser einer hohen Reibung unterliegt, was zu einer Behinderung beim Aufschieben des Katheters auf den Führungsdraht führt.

Zum anderen ist der erörterte Katheter nur zum Einsatz mit Führungsdraht gedacht, während für Katheter zum Einsatz nur mit Mandrin andere Ausführungsformen gewählt werden. Insoweit werden im Stand der Technik grundsätzlich Katheter zur Verfügung gestellt, die entweder zum Einsatz mit Führungsdraht oder mit Mandrin konzipiert sind. Dies bedingt eine Verdoppelung des Herstellungs- und Lagerhaltungsaufwandes bei im Übrigen oft baugleichen Kathetern. Der entsprechende Mehraufwand schlägt sich auch bei der Logistik und Lagerhaltung in der klinischen Praxis nieder.

Ausgehend von den geschilderten Problemen des Standes der Technik, liegt der Erfindung die Aufgabe zugrunde, einen Katheter der eingangs geschilderten Art so weiterzubilden, dass er universell mit Führungsdraht oder Mandrin eingesetzt werden kann.

Diese Aufgabe wird durch die im Kennzeichnungsteil des Anspruches 1 angegebenen Merkmale gelöst.

Gemäß der Erfindung ist ein Anschlag proximal vor der Dichteinheit angeordnet, der mit einem Gegenstück an einem in das Lumen einführbaren Mandrin kooperiert. Dieser Anschlag dient wiederum einerseits als Widerlager für den Mandrin, andererseits verhindert er, dass der Mandrin die Dichteinheit beim Arbeiten ohne Führungsdraht durchstößt. Damit wird eine zuverlässige Abdichtung des Katheterlumens für diesen Anwendungsfall gewährleistet.

Bevorzugte Ausführungsformen der Erfindung betreffen ferner die Ausbildung des Anschlages als ringschulterförmige Durchmesserstufe und das Zusammenwirken des proximalen Anschlages mit einer Kugel am Gegenstück des führungsdrahtlosen Mandrins.

Weitere Merkmale, Einzelheiten und Vorteile der Erfindung ergeben sich aus der nachfolgenden Beschreibung, in der ein Ausführungsbeispiel des Erfindungsgegenstandes anhand der beigefügten Zeichnungen näher erläutert wird. Es zeigen:
- Fig. 1: einen Axialschnitt durch das distale Ende eines Katheters mit eingesetztem Führungsdraht, und
- Fig. 2: einen Teilschnitt analog Fig. 1 mit einem eingesetzten Mandrin ohne Führungsdraht.

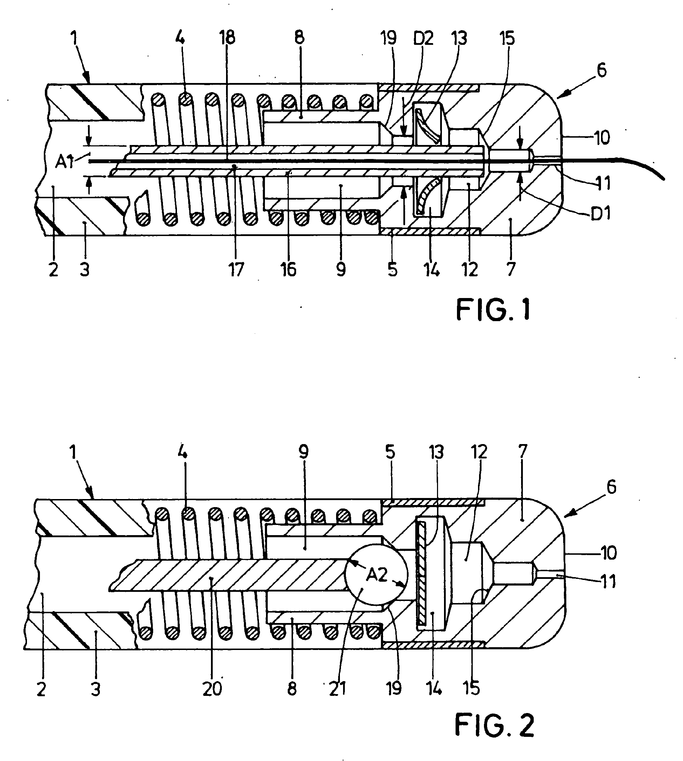

Der in Fig. 1 und 2 ausschnittsweise mit seinem distalen Ende gezeigte Katheter weist einen Katheterschaft 1 auf, in dem ein axial durchgehendes, zentrales Lumen 2 angelegt ist. In der Wand 3 des Katheterschaftes läuft eine gewendelte Anschlussleitung 4 für eine Ringelektrode 5, die in einem das distale Ende 6 des Katheters bildenden, isolierenden Kopfkörper 7 angeordnet ist.

Der Kopfkörper 7 weist in proximaler Richtung einen Ringansatz 8 auf, auf dem einerseits die gewendelte Anschlussleitung 4 mit ihrem distalen Ende sitzt und in dem andererseits das zentrale Lumen 2 durch die Innenöffnung 9 fortgesetzt ist.

In der distal weisenden Stirnfläche 10 des Kopfkörpers 7 ist zentral eine Schleusenöffnung 11 eingebracht, die über einen zentralen Zugangskanal 12 im Kopfkörper 7 vom Lumen 2 und der Innenöffnung 9 her zugänglich ist. Proximal vor der Schleusenöffnung 11 ist in diesem Zugangskanal 12 eine als flexible Scheibe mit einer zentralen, expandierbaren Öffnung ausgebildete Dichteinheit 13 angeordnet, die in einem Ringraum 14 des Zugangskanals 12 liegt. Zwischen Dichteinheit 13 und Schleusenöffnung 11 ist im Zugangskanal 12 ein erster, distaler Anschlag 15 vorgesehen, der durch eine entsprechende Durchmesserstufe gebildet ist. Der Zugangskanal 12 als Fortsetzung des Lumens 2 verringert sich dort auf einen Innendurchmesser D1, der kleiner ist, als der Außendurchmesser A 1 einer Mandrinhülse 16, die im Lumen 2 verläuft, die Dichteinheit 13 durchstößt und durch die vorstehenden Dimensionsverhältnisse am distalen Anschlag 15 gegen ein weiteres Ausschieben aus dem Lumen 2 blockiert ist.

In der zentralen Hülsenöffnung 17 ist schließlich ein Führungsdraht 18 quasi frei und ohne relevante Reibung relativ zur Mandrinhülse 16 und damit zum Katheterschaft 1 verschiebbar. Insoweit kann der Katheter nach Einführen des Führungsdrahtes 18 in das gewünschte Herzgefäß problemlos über den Führungsdraht 18 geschoben werden. Durch die Dichteinheit 13 ist das Lumen 2 gegen eintretendes Blut sauber abgedichtet.

Wie ferner aus den beiden Fig. 1 und 2 hervorgeht, ist an der Basis des Ringansatzes 8 ein zweiter, proximaler Anschlag 19 in Form einer Durchmesserstufe vorgesehen, dessen Innendurchmesser D2 deutlich größer als der Innendurchmesser D1 des ersten, distalen Anschlages 15 ist. Insbesondere ist dieser Innendurchmesser D2 größer als der Außendurchmesser A1 der Mandrinhülse 16, sodass der Anschlag 19 ein Durchschieben der Mandrinhülse 16 durch die Dichteinheit 13 bis zum distalen Anschlag 15 nicht behindert.

Soll nun mit einem herkömmlichen Mandrin ohne Führungsdraht 18 gearbeitet werden, so kann die Mandrinhülse 16 entfernt und ein in Fig. 2 erkennbarer Mandrin 20 eingeschoben werden, der über einen Kugelkopf 21 an seinem distalen Ende als Gegenstück gegen den Anschlag 19 fährt. Der Außendurchmesser A2 des Kugelkopfes 21 ist größer als der Innendurchmesser D2 des proximalen Anschlags 19. Dadurch wird verhindert, dass der Mandrin 20 durch die Dichteinheit 13 hindurchgeschoben wird. Das Lumen 2 bleibt sauber abgedichtet.

Durch die Einbettung der Anschlussleitung 4 in die Wand 3 des Katheterschaftes 2 sind der Mandrin 20 beziehungsweise die Mandrinhülse 16 und der Führungsdraht 18 von der Anschlussleitung 4 elektrisch isoliert.

## Patentansprüche

1. Katheter zur kardiovaskulären Anwendung umfassend
- einen langgestreckten Katheterschaft (1),
- ein Lumen (2) im Katheterschaft (1),
- eine Schleusenöffnung (11) am distalen Ende (6) des Katheters, aus der ein Führungsdraht (18) herausführbar ist,
- eine proximal vor der Schleusenöffnung (11) angeordnete Dichteinheit (13) zur Abdichtung des Lumens (2), und
- einen im Lumen (2) proximal vor der Dichteinheit (13) angeordneten proximalen Anschlag (19) mit einem Innendurchmesser (D2), der mit einem Gegenstück (21) an einem in das Lumen (2) einführbaren Mandrin (20) kooperiert
**dadurch gekennzeichnet, dass**
der Innendurchmesser (D2) des Anschlags (19) ein Durchschieben einer Mandrinhülse (16) durch die Dichteinheit (13) nicht behindert.

2. Katheter nach Anspruch 1, **dadurch gekennzeichnet, dass** der proximale Anschlag (19) durch eine ringschulterförmige Durchmesserstufe gebildet ist.

3. Katheter nach Anspruch 1 oder 2, **dadurch gekennzeichnet, dass** die Dichteinheit (13) in einem distal des Lumens (2) liegenden Zugangskanal (12) angeordnet ist.

4. Katheter nach Anspruch 3, **dadurch gekennzeichnet, dass** der Zugangskanal (12) distal des Dichtelementes (13) einen distalen Anschlag (15) mit einem Innendurchmesser (D1) aufweist, der kleiner ist als der Außendurchmesser (A1) der Mandrinhülse (16) und der ein weiteres Ausschieben der Mandrinhülse (16) aus dem Lumen (2) verhindert.

5. Katheter nach Anspruch 1 oder 2, **dadurch gekennzeichnet, dass** das Gegenstück am Mandrin (20) eine Kugel (21) ist, deren Durchmesser (A2) größer als der Innendurchmesser (D2) des proximalen Anschlags (19) ist.

## Claims

1. A catheter for cardiovascular use comprising
- an elongate catheter shaft (1),
- a lumen (2) in the catheter shaft (1),
- a lock opening (11) at the distal end (6) of the catheter, out of which a guide wire (18) may be led,
- a seal unit (13) situated proximally in front of the lock opening (11) to seal the lumen (2), and
- a proximal stop (19), situated in the lumen (2) proximally in front of the seal unit (13), having an internal diameter (D2), which cooperates with a counterpart (21) on a mandrin (20) insertable into the lumen (2)
**characterized in that** the internal diameter (D2) of the stop (19) does not obstruct a mandrin sleeve (16) from being pushed through the seal unit (13).

2. The catheter according to Claim 1, **characterized in that** the proximal stop (19) is formed by a diameter step in the form of a ring shoulder.

3. The catheter according to Claim 1 or 2, **characterized in that** the seal unit (13) is situated in an access channel (12) lying distally of the lumen (2).

4. The catheter according to Claim 3, **characterized in that** the access channel (12) has a distal stop (15) distally of the seal element (13), having an internal diameter (D1) which is less than the external diameter (A1) of the mandrin sleeve (16) and prevents the mandrin sleeve (16) from being pushed further out of the lumen (2).

5. The catheter according to Claim 1 or 2, **characterized in that** the counterpart on the mandrin (20) is a ball (21), whose diameter (A2) is greater than the internal diameter (D2) of the proximal stop (19).

## Revendications

1. Cathéter pour l'application cardiovasculaire comprenant
- une tige de cathéter (1) étirée en longueur,
- un lumen (2) dans la tige de cathéter (1),
- une ouverture de sas (11) sur l'extrémité (6) distale du cathéter, de laquelle un fil de guidage (18) peut être sorti,
- une unité d'étanchéité (13) disposée à proximité avant l'ouverture de sas (11) pour l'étanchéité du lumen (2), et
- une butée (19) proximale disposée dans le lumen (2) à proximité avant l'unité d'étanchéité (13) avec un diamètre intérieur (D2), qui est coopératif avec une pièce inverse (21) sur un mandrin (20) introduit dans le lumen (2),
**caractérisé en ce que**
le diamètre intérieur (D2) de la butée (19) n'empêche pas un coulissement d'une douille de mandrin (16) à travers l'unité d'étanchéité (13).

2. Cathéter selon la revendication 1, **caractérisé en ce que** la butée proximale (19) est formée par un niveau de diamètre en forme d'épaulement annulaire.

3. Cathéter selon la revendication 1 ou 2, **caractérisé en ce que** l'unité d'étanchéité (13) est disposée dans un canal d'accès (12) situé au plan distal du lumen (2).

4. Cathéter selon la revendication 3, **caractérisé en ce que** le canal d'accès (12) présente au plan distal de l'élément d'étanchéité (13) une butée (15) distale avec un diamètre intérieur (D1) qui est inférieur au diamètre extérieur (A1) de la douille de mandrin (16) et qui empêche une nouvelle sortie de la douille de mandrin (16) du lumen (2).

5. Cathéter selon la revendication 1 ou 2, **caractérisé en ce que** la pièce inverse est sur le mandrin (20) une bille (21), dont le diamètre (A2) est supérieur au diamètre intérieur (D2) de la butée proximale (19).
